# EUROPÄISCHE PATENTSCHRIFT

(11) **EP 0 722 344 B1**
(45) Veröffentlichungstag und Bekanntmachung des Hinweises auf die Patenterteilung: **16.01.2002**
(21) Anmeldenummer: 94928864.1
(22) Anmeldetag: 06.10.1994
(51) Int. Cl.: A61L 2/18, A61K 35/16

(54) **VERFAHREN ZUR VIRUSINAKTIVIERUNG IN GEGENWART EINES POLYETHERS UND EINES AGENS**
PROCESS FOR VIRUS DEACTIVATION IN THE PRESENCE OF A POLYETHER AND AN AGENS
PROCEDE PERMETTANT D'INACTIVER UN VIRUS EN PRESENCE D'UN POLYETHER ET D'UN AGENT

(30) Priorität: 06.10.1993 DE 4334087; 27.09.1994 DE 4434538
(43) Veröffentlichungstag der Anmeldung: 24.07.1996
(73) Patentinhaber: Baxter Aktiengesellschaft, 1221 Wien (AT)
(72) Erfinder: EIBL, Johann, A-1180 Wien (AT); DORNER, Friedrich, A-1230 Wien (AT); BARRETT, Noel, A-3400 Klosterneuburg/Weidling (AT)
(74) Vertreter: Kolb, Helga, Dr. Dipl.-Chem.
(86) Internationale Anmeldenummer: EP9403298
(87) Internationale Veröffentlichungsnummer: WO9509657

(56) Entgegenhaltungen:
- EP-A- 0 083 999
- EP-A- 0 099 445
- EP-A- 0 177 836
- EP-A- 0 530 173
- FR-A- 2 339 621
- US-A- 4 315 919
- 'RÖMPP CHEMIE LEXIKON', 1995, GEORG THIEME VERLAG, STUTTGART

## Beschreibung

Die Erfindung umfaßt ein Verfahren zur Herstellung eines biologischen Präparats nach Anspruch 1, welches nachweislich potentiell vorhandene Viren inaktiviert.

Plasmaproteine sind Proteine, die aus menschlichem oder tierischem Blut bzw. Plasma gewonnen werden können. Die Plasmaproteine sind als pharmazeutische Präparation zur therapeutischen, prophylaktischen oder diagnostischen Anwendung bestimmt. Solche Präparationen können Enzyme, Proenzyme einschließlich Gerinnungsfaktoren, Enzymcofaktoren, Enzyminhibitoren, Immunglobuline, Albumin, Plasminogen, Fibrinogen, Fibronectin, t-PA, Urokinase, Prourokinase oder Plasma enthalten. Als Plasmaproteine werden ebenso rekombinante Polypeptide verstanden, die aufgrund ihrer Eigenschaften den genannten Plasmaproteinen äquivalent sind.

Unter biologischen Präparaten werden pharmazeutisch anwendbare Präparate verstanden, die biologischen Ursprungs sind, d.h. aus natürlichen Quellen isoliert oder aus Zellkulturen gewonnen bzw. rekombinant hergestellt sind. Ein biologisches Präparat enthält beispielsweise ein Plasmaprotein oder eine Vaccine, insbesondere ein virales Antigen.

Zu den biologischen Präparaten zählen aber auch Immunglobulinpräparate, monoklonale Antikörper oder deren Fragmente, Überstände von Zellkulturen oder Ascitesflüssigkeit von Mäusen.

Bei der Verabreichung eines biologischen Präparates, insbesondere eines Plasmaprotein-haltigen Präparates besteht ein Infektionsrisiko durch potentiell vorhandene infektiöse Agenzien, wie Hepatitis- oder AIDS-Viren. Das Herstellungsverfahren dieser Präparate muß daher geeignete Inaktivierungsmaßnahmen umfassen.

Unter infektiösen Agenzien werden Krankheitserreger verstanden, die von einem Organismus auf einen anderen Organismus übertragen werden können, beispielsweise Viren oder Prionen.

Es liegt eine umfangreiche Literatur vor, die sich mit der Inaktivierung von infektiösen Agenzien in pharmazeutischen Präparationen befaßt.

Als eine der wirksamsten Methoden zur Inaktivierung von Viren gilt das Erhitzen von Plasmaproteinen in Lösung. Es ist bekannt, daß eine virussichere albuminhaltige Präparation durch Erhitzen einer wäßrigen Albuminlösung bei einer Temperatur von 60°C während 10 h hergestellt werden kann. Die biologische Aktivität des Albumins ist dabei nicht beeinträchtigt, da Albumin ein relativ stabiles Plasmaprotein ist.

Durch den Zusatz von Ammoniumsulfat ist im Stand der Technik eine Erhöhung der Virusinaktivierungskapazität einer Wärmebehandlung von Blutprodukten in Lösung beschrieben (EP-124 506). Dabei ist das Problem der gleichzeitigen Inaktivierung von Plasmaproteinen bekannt, weshalb vorgeschlagen wird, Protein-stabilisierende Substanzen, wie Glycin, zuzusetzen. Eine erwünschte Stabilisierung der Plasmaproteine bedeutet aber gleichzeitig eine unerwünschte Stabilisierung der infektiösen Agenzien. Das Bestreben geht also dahin, eine Infektivität der Präparation auszuschließen und gleichzeitig ihre biologische Aktivität weitgehend zu erhalten.

Ein Zusatz von Stabilisatoren zu Plasmaprotein-haltigen Lösungen ist beispielsweise aus EP-A-292 003 bekannt. Als Stabilisatoren werden Saccharide oder Zuckeralkohole und neutrale Salze, wie Acetate, Phosphate und Sulfate zugesetzt. Salze haben hier eine stabilisierende Wirkung.

Eine virusinaktivierende Wirkung von Salzen ist hingegen in WO-A-90/07524 beschrieben. Antikörper gegen Protein C sind bei 22°C mindestens 2 h in Gegenwart von mindestens 2,6 M Guanidin oder 2 M Kaliumthiocyanat stabil. Eine solche Behandlung wird auch vorgeschlagen, um potentiell vorhandene Viren zu inaktivieren. Die genannten Verbindungen haben aufgrund ihres chaotropen Verhaltens nicht nur inaktivierende Wirkung gegenüber Viren, sondern auch gegenüber Proteinen. Deshalb ist es beachtlich, daß die Antikörper bei Raumtemperatur eine gewisse Zeit mit diesen Verbindungen inkubiert werden können, ohne ihre Affinität zu Protein C zu verlieren.

Aus WO-A-90/15613 ist ein Verfahren zur Inaktivierung von Viren durch Zusatz von Natriumthiocyanat zu Plasmaprotein-haltigen Lösungen bekannt. Um das Plasmaprotein nicht zu denaturieren wird darauf geachtet, daß die Behandlung möglichst bei 4°C und während einer kurzen Behandlungsdauer erfolgt.

Thiocyanate werden darüber hinaus als Elutionsmittel im Rahmen einer Immunaffinitätschromatographie eingesetzt. Im Anschluß an die Elution erfolgt unmittelbar die Entfernung des Thiocyanates, um eine Schädigung des gereinigten Protein. zu verhindern (vgl. US-A-5 055 557).

Die EP-A-0 177 836 beschreibt die Inaktivierung von Vinen in biologischen Präparaten durch Polyether Zusammen mit Glycin, Albumin, Mannitol, Na - Chlorid oder Na-Acetat.

Die EP-A-0 530 173 beschreibt die Verwendung eines chaotropen Agens mit einem Detergens.

Die Erfindung stellt sich die Aufgabe, ein Verfahren zur Herstellung von biologischen Präparate zur Verfügung zu stellen, die aufgrund ihres Herstellungsverfahrens frei von infektiösen Agenzien sowie weitgehend frei von Denaturierungsprodukten ist.

Die vorstehende Aufgabe wird gemäß der Erfindung durch das Verfahren nach Anspruch 1 gelöst:

Polyether umfassen gemäß der Erfindung auch Polyhydroxyether, wie Polyalkylenglykol, und insbesondere Polyethylenglykol und Polypropylenglykol.

Eine bevorzugte Ausführungsform ist dadurch gekennzeichnet, daß man in Stufe
(a) als Polyether ein niedermolekulares Polyethylenglykol ausgewählt aus der Gruppe PEG 200, PEG 300, PEG 400, PEG 600, PEG 900 und PEG 1000 zugibt,
(b) die Inaktivierung von infektiösen Agenzien in Gegenwart des Polyethylenglykols durchführt, und
(c) das Polyethylenglykol entfernt.

In einer bevorzugten Ausführungsform erfolgt in Stufe (b) die Inaktivierung von infektiösen Agenzien in Gegenwart des Polyethers durch eine physikalische, physikalisch-chemische oder chemische Behandlung mit der Maßgabe, daß eine Detergens-Behandlung ausgenommen ist.

Gegenstand der Erfindung ist ein Verfahren gemäß Anspruch 1 zur Herstellung eines biologischen Präparats.

Bevorzugte Ausgestaltungen dieser Verfahren sind Gegenstand der Ansprüche 2 bis 17.

Es wurde überraschenderweise gefunden, daß der Zusatz eines Polyethers, wie z.B. Polyethylenglykol, in einer Konzentration von 5 bis 30 Gew.-%, einen unerwartet positiven Effekt auf eine Inaktivierungsbehandlung zur Beseitigung von Infektiosität ausübt. Dadurch können die Bedingungen der Inaktivierungsbehandlung so gewählt werden, daß die biologische Aktivität der Plasmaproteine weitgehend erhalten bleibt.

Die Konzentration des Polyethers wird so gewählt, daß keine Fällungsreaktionen verursacht werden, auch bei Zusatz von chaotropen Salzen wird die Behandlung vorzugsweise so durchgeführt, daß Proteine nicht gefällt werden. Die Präzipitation von Proteinen birgt nämlich die Gefahr in sich, daß infektiöse Partikel im Präzipitat eingeschlossen sind. Dadurch werden die infektiösen Partikel einer Inaktivierungsbehandlung schlecht zugänglich. In Abwesenheit eines Präzipitats ist daher keine verzögerte Inaktivierung von infektiösen Agenzien zu beobachten. Ebenso ist es möglich, die Temperätur einer Wärmebehandlung bei gleichbleibender Effektivität herabzusetzen.

Die Behandlung zur Inaktivierung von infektiösen Agenzien umfaßt vorzugsweise eine Wärmebehandlung, insbesondere bei einer Temperatur von 20 bis 60°C, vorzugsweise im Bereich von 25 bis 45°C.

Als geeigneter Polyether wird vorzugsweise Polyethylenglykol, und besonders bevorzugt ein niedermolekulares Polyethylenglykol verwendet. Als besonders geeignete Polyethylenglykole sind hier solche zu nennen, die aus der Gruppe PEG 200, PEG 300, PEG 400, PEG 600, PEG 900 und PEG 1000 ausgewählt werden. Zur Entfernung des Polyethers eignen sich eine Reihe von bekannten Maßnahmen. Das Plasmaprotein wird aus der behandelten Lösung vorteilhafterweise durch Präzipitation oder Adsorption, vorzugsweise durch Chromatographie, entfernt. Der Polyether verbleibt hingegen in Lösung und wird so vom Plasmaprotein abgetrennt. Die Entfernung des Polyethers erfolgt vollständig bzw. bis zu einem physiologisch akzeptablen Gehalt im verabreichungsfertigen Präparat.

Gemäß der Erfindung kann vorgesehen sein, daß nach Zugabe eines Polyethers zu einer das Plasmaprotein enthaltenden Lösung dieselbe lyophilisiert wird, so daß die anschließende Behandlung des Plasmaproteins zur Inaktivierung von infektiösen Agenzien in Gegenwart des Polyethers im festen Zustand als Lyophilisat erfolgt. Diese Behandlung stellt eine physikalisch-chemische oder eine chemische Behandlung dar. Dazu zählt z.B. auch eine Behandlung in Gegenwart von viruziden Substanzen, gegebenenfalls kombiniert mit einer Strahlenbehandlung oder Hitzebehandlung.

Erfindungsgemäß erfolgt die Inaktivierung von infektiösen Agenzien durch Behandlung einer Lösung oder eines Lyophilisats der Präparation in Gegenwart von einem chaotropen Agens. Es hat sich herausgestellt, daß durch die Kombination eines chaotrop wirksamen Salzes, wie z.B. Thiocyanat, mit einem Polyether, wie z.B. Polyethylenglykol, ein synergistischer Effekt auf die Inaktivierung von Viren beobachtet wird, wobei die biologische Aktivität der Präparation im wesentlichen erhalten bleibt. So werden auch resistente Viren, wie Vaccinia-Virus oder Parvovirus, im Vergleich zu einer Behandlung mit Thiocyanat alleine, wesentlich rascher und bei geringeren Konzentrationen an Thiocyanat inaktiviert. Die Reduktion der Thiocyanat-Konzentration und der Behandlungsdauer wirkt sich wiederum vorteilhaft auf die biologische Aktivität des Präparates aus.

Als chaotropes Agens wird ein Thiocyanat, Harnstoff oder ein Guanidiniumsalz verwendet. Unter den letzteren sind insbesondere Natrium-, Ammonium- oder Kaliumthiocyanat zu nennen. Als Guanidiniumsalz ist insbesondere Guanidiniumhydrochlorid bevorzugt.

Die chaotropen Agenzien werden in Konzentrationen von ca. 0,1 bis 2 M verwendet. Auch hier wird, wie bei der bereits vorstehend beschriebenen Verfahrensvariante, die Behandlung in Gegenwart von chaotropen Salzen unter Bedingungen durchgeführt, bei denen Proteine nicht gefällt werden.

Mit dem erfindungsgemäßen Verfahren ist es erstmals möglich, labile Plasmaproteine, d.h. leicht denaturierbare Proteine, in Gegenwart von chaotropen Salzen in Lösung zu behandeln und die biologische Aktivität der Plasmaproteine im wesentlichen zu erhalten.

Der Zusatz eines Polyethers und eines chaotropen Agens während der Virusinaktivierung ermöglicht also die Herstellung einer pharmazeutischen Präparation, die Plasmaproteine enthält, welche weitgehend frei von Denaturierungsprodukten sind.

Die Entfernung des chaotropen Agens erfolgt ebenso auf an sich bekannte Weise bis zu einem Gehalt, der die physiologische Verträglichkeit des Präparates nicht beeinträchtigt. Vorzugsweise soll dieser Gehalt unterhalb der Nachweisgrenze liegen. Zur Entfernung von chaotrop wirksamen Salzen kann die plasmaproteinhaltige Lösung dialysiert bzw. ultrafiltriert werden. Gleichzeitig mit dieser physikalischen Behandlung werden potentiell vorhandene infektiöse Partikel abgetrennt. Andererseits kann das Plasmaprotein durch Präzipitation und/oder Adsorption, vorzugsweise durch Chromatographie, vom chaotropen Agens abgetrennt werden.

In einer der vorstehend genannten zweckmäßigen erfindungsgemäßen Ausführungsformen wird in Stufe (b) eine Detergensbehandlung ausgenommen; unter einer solchen Detergensbehandlung ist eine Behandlung mit für eine Virusinaktivierung gemäß dem Stand der Technik üblicherweise verwendeten Detergentien im engeren Sinne zu verstehen, d.h. also mit z.B. Tensiden, wie die für derartige Zwecke eingesetzten oberflächenaktiven Mittel, z.B. insbesondere Polyoxyethylenderivate der Sorbitanester (Polysorbat), die unter dem Warenzeichen "Tween" erhältlich sind. Solche Detergentien werden im Zusammenhang mit einer Virusinaktivierung, zumindest in Kombination mit anderen Maßnahmen, z.B. beschrieben in EP-A-479597 (wo insbesondere Tween 80 verwendet wird), US-A-4764369 (nach der die Virusinaktivierung mit Di- oder Trialkylphosphat in Kombination mit Detergentien durchgeführt wird), und EP-B-0050061 (worin die Virusinaktivierung und Verringerung oder Beseitigung von Substanzen, die unerwünschte Wirkungen, z.B. Pyrogenizität besitzen, durch Zugabe eines amphiphilen Mittels erfolgt, das z.B. ein Gallensäuresalz, ausgewählt aus Natriumcholat und Natriumdeoxycholat, oder ein nichtionisches Tensid ausgewählt aus den polyoxyethylierten Derivaten der partiellen Ester von C₁₂-C₂₂-Fettsäuren und Anhydriden ist).

Unter den erfindungsgemäß ausgenommenen Detergentien sind insbesondere zu verstehen:

Nicht-ionische Detergentien, Polyoxyethylenderivate von Fettsäuren, partielle Ester von Sorbit(ol)anhydriden, wie z.B. "Tween 80", "Tween 20" und "Polysorbat 80", und nicht-ionische öllösliche Detergentien, wie sie unter dem Warenzeichen "Triton X-100" (oxyethyliertes Alkylphenol) vertrieben werden, sowie außerdem Natriumdeoxycholat, und sogenannte "Zwittergents", d.h. synthetische zwitterionische Detergentien, die als "Sulfobetaine" bekannt sind, wie z.B. N-Dodecyl-N,N-dimethyl-2-ammonio-1-ethansulfonat, und ähnliche Substanzen, oder nicht-ionische Detergentien, wie z.B. Octyl-beta-D-glucopyranosid.

Im allgemeinen sind nicht-ionische oberflächenaktive oxyethylierte Alkylphenole Polyoxyethylensorbitanfettsäureester, Polyoxyethylensäuren und Polyoxyethylenoxypropylenfettsäuren. Spezifische Beispiele davon sind die folgenden:
Alkylphenoxypolyethoxy (30) ethanol
Polyoxyethylen (2) sorbitanmonolaurat
Polyoxyethylen (20) sorbitanmonopalmitat
Polyoxyethylen (20) sorbitanmonostearat
Polyoxyethylen (20) sorbitantristearat
Polyoxyethylen (20) sorbitanmonooleat
Polyoxyethylen (20) sorbitantrioleat
Polyoxyethylen (20) palmitat
Polyoxyethylen (20) laurylether
Polyoxyethylen (20) cetylether
Polyoxyethylen (20) stearylether
Polyoxyethylen (20) oleylether
Polyoxyethylen (20) hydriertes Castoröl
Polyoxyethylen (20) oxypropylenmonostearat.

Amphiphile oberflächenaktive Mittel, die sowohl hydrophile wasserlösliche als auch hydrophobe wasserunlösliche Gruppen enthalten, und die häufig in anionische, kationische, ampholytische und nicht-ionische oberflächenaktive Mittel eingeteilt werden; Beispiele für solche amphiphile, erfindungsgemäß ausgeschlossene Detergentien sind:

Anionische Mittel:
Sulfatiertes oxyethyliertes Alkylphenol (Triton W-30® ); sulfatierter Lauryletheralkohol;
Natriumdodecylbenzolsulfonat (Nacconol NR® );
Natrium 2-sulfoethyloleat (Igepon A® );
Natrium N-methyl-N-oleylethanol sulfonat (Igepon T® );
Natriumdodecylsulfat;
Natriumcholat;
Natriumdeoxycholat;
Natriumdodecylsulfonat;
Natriumdodecyl-N-sarconisat.

Kationische Mittel:
Dodecyldimethylbenzylammoniumchlorid (Triton K-60® );
oxyethylierte Amine (Ethomeen® );
Cetyltrimethylammoniumbromid;
Tetradecylammoniumbromid;
Dodecylpyrimidiniumchlorid;
Hexadecyltrimethylammoniumchlorid.

Ampholytische Mittel:
Dodecyl beta-alanin;
N-Dodecylaminoethansulfonsäure;
Palmitoyllysolecithin;
Dodecyl-N-betain.

Oxyethylierte Alkylphenole (Triton X-100® ), partielle Ester von C₁₂-C₂₂-Fettsäuren (z.B. Laurin-, Palmitin-, Stearin- und Ölsäuren) und Hexitanhydride (z.B. Hexitane und Hexide) (Spans), wie sie z.B. in den US-Patenten 2232820, 2232821, 2303432 beschrieben werden; polyoxyethylierte Derivate dieser partiellen Ester, die durch Addition von Polyoxyethylenketten an nicht-esterifizierte Hydroxylgruppen erhalten werden (Tween® , z.B. Tween 80® oder Polysorbat 80® ) wie z.B. in US-Patent 2380166 beschrieben; partielle polyoxyethylierte Fettsäureester (Myrj45® ); Polyoxyethylenfettsäurealkoholether (Brij® ).

Unter den ausgeschlossenen oxyethylierten Alkylphenolen (Triton X), sind insbesondere zu nennen die der Formel RC₆H₄(OC₂H₄)ₙOH, worin R Octyl oder Nonyl ist und n mindestens 3 bedeutet, wie z.B. Octylphenoxyethanol; solche oberflächenaktive Mittel werden unter dem Warenzeichen "Triton X", z.B. Triton x-100, Triton X-165, Triton X-205, Triton X-305, Triton X-405 und Triton N-100 vertrieben.

Als weitere Detergentien, die gemäß der erfindungsgemäßen zweckmäßigen Ausführungsform ausgenommen sind, sind die amphiphilen Detergentien: Gallensäuresalze, wie z.B. Natriumcholat und Natriumdeoxycholat.

Die Präparation zeichnet sich überraschenderweise vor allem durch ein sehr geringes Ausmaß an Denaturierung aus. Die biologische Aktivität des Plasmaproteins nach Inaktivierung von infektiösen Agenzien ist - im Vergleich zur Aktivität von der Inaktivierungsbehandlung - zu mindestens 50 %, vorzugsweise mindestens 80 %, am meisten bevorzugt zu mindestens 90 % erhalten.

Die biologische Aktivität der Präparation wird im Falle eines Faktors der Gerinnung, Fibrinolyse oder Thrombolyse, oder dessen Inhibitor gemessen durch seinen Einfluß auf die enzymatische Reaktion im Rahmen der Blutgerinnung. Die biologisch Aktivität eines Immunglobulins kann nach der Auftrennung des Präparates mittels HPLC-Chromatographie beurteilt werden. Etwaige Denaturierungsprodukte bzw. Aggregate werden so von Immunglobulin-monomeren bzw. -dimeren abgetrennt und quantitativ bestimmt.

Der positive Effekt auf die Inaktivierung von infektiösen Agenzien durch Polyether, wie z.B. Polyethylenglykol, war für den Fachmann überraschend. Es war allgemein bekannt, daß Substanzen, die Plasmaproteine stabilisieren, auch eine stabilisierende Wirkung auf Viren ausüben. In diesem Zusammenhang sei z.B. auf eine Veröffentlichung von B. Horowitz et al. in Transfusion, 25, 523-527 (1985) verwiesen. Danach war in Gegenwart eines Polyethers eine verschlechterte Inaktivierungskinetik zu erwarten.

Das erfindungsgemäße Verfahren wird so lange durchgeführt, daß potentiell vorhandene Viren aus der Gruppe der großen membranumhüllten RNA-Viren, kleinen membranumhüllten RNA-Viren und der membranumhüllten DNA-Viren vollständig inaktiviert werden. Dies kann durch Versuche mit Modellviren bestätigt werden. Die Bedingungen des erfindungsgemäßen Verfahrens werden so gewählt, daß ein dem biologischen Präparat zugesetztes Virus aus jeder Gruppe durch das erfindungsgemäße Verfahren inaktiviert wird, so daß der Virustiter unter der Nachweisgrenze liegt. Als Modellvirus eignen sich vor allem HIV, FSME-Virus und Pseudorabies-Virus (PSR).

Das erfindungsgemäße Verfahren eignet sich insbesondere zur Inaktivierung von Hepatitis-Viren, vor allem von Hepatitis B, Hepatitis C und non-A-non-B-Hepatitisviren sowie von Retroviren, vor allem von AIDS-Viren.

Die Erfindung wird durch die nachfolgenden Beispiele noch näher erläutert:

### 1. Inaktivierung von Modellviren in einer Gammaglobulin-haltigen Lösung mit Thiocyanat in Gegenwart von Polyethylenglykol

Eine 10%ige Lösung, enthaltend ein i.m.verträgliches Gammaglobulin wurde hergestellt durch eine Plasmafraktionierung nach Cohn. Die Lösung wurde mit einer Suspension, enthaltend HIV-1, FSME-Virus oder PSR-Virus versetzt. Der Lösung wurde Ammoniumthiocyanat bis zu einer Konzentration von 0,3 M und PEG 200 bis zu einem Gehalt von 10 Gew.-% zugesetzt. Anschließend wurde auf 30°C erwärmt und der jeweilige Virustiter nach 0,1, 3, 6 und 10 h bestimmt. Die Ergebnisse sind in der nachfolgenden Tabelle angeführt. Als Kontrollwert diente der Virustiter in der verwendeten Virussuspension. Der Virustiter zur Zeit der Behandlungsdauer 0 ist jeweils in den Tabellen aufgeführt.

Mittels HPLC konnte festgestellt werden, daß es durch die Inaktivierungsbehandlung zu keiner Aggregatbildung gekommen ist.

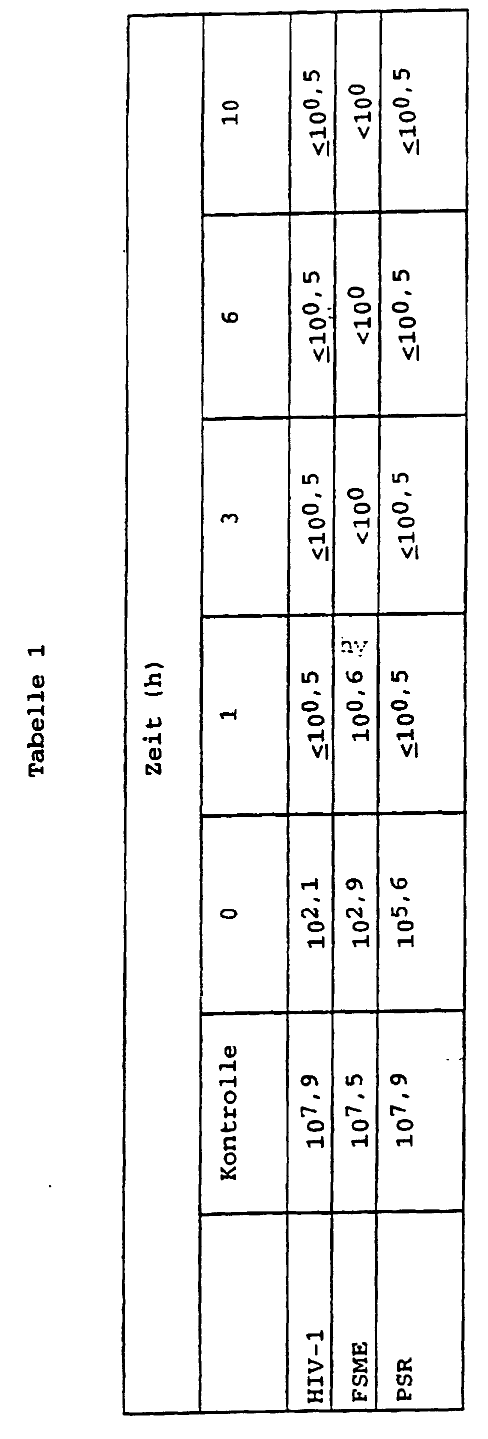

### 2. Vergleichsbeispiel mit jeweils nur Ammoniumthiocyanat bzw. Polyethylenglykol

Der Gammaglobulin-haltigen Lösung aus Beispiel 1 wurde in einem Ansatz Ammoniumthiocyanat in einer Konzentration von 0,3 M zugegeben. In einem weiteren Ansatz wurde der Gammaglobulin-haltigen Lösung aus Beispiel 1 PEG 200 bis zu einem Gehalt von 10 bzw. 30 % zugegeben. Die Lösungen wurden mit einer HIV-1-haltigen Suspension versetzt und auf einer Temperatur von 30°C gehalten. Der Virustiter wurde 0, 0,5, 1, 1,5, 2, 3, 6 und 10 h bestimmt. Die Ergebnisse sind in der nachfolgenden Tabelle angeführt.

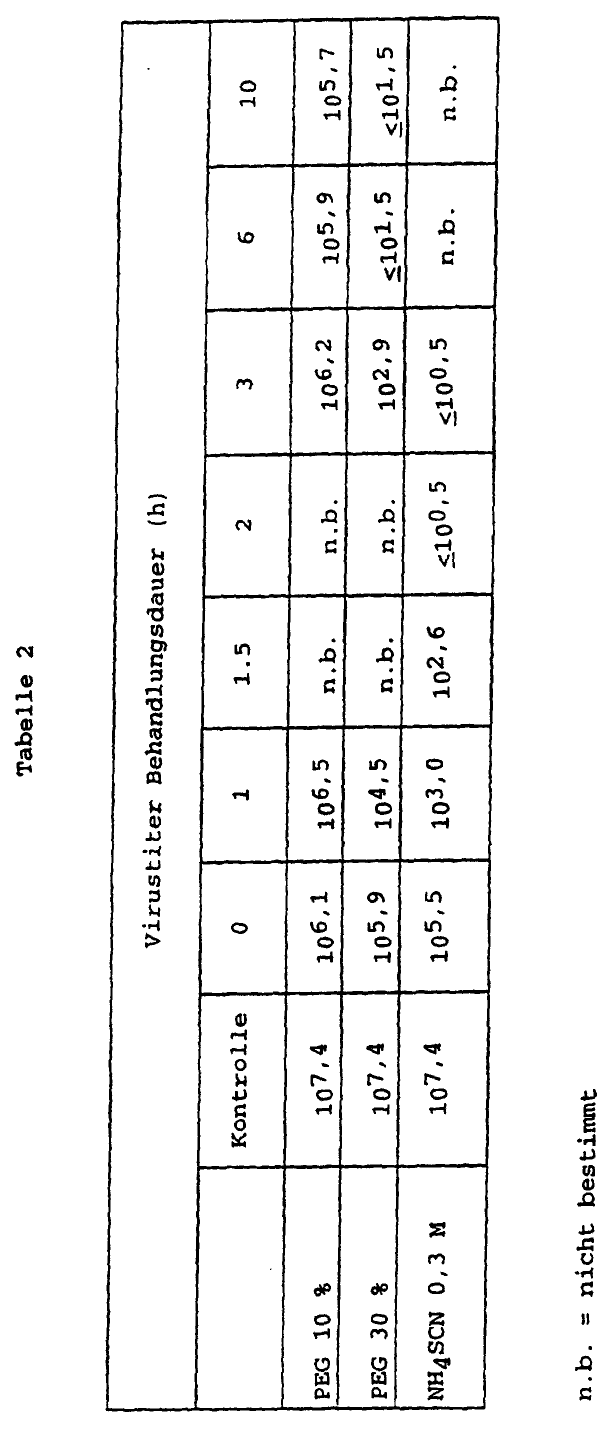

Aus Tabelle 2 ist deutlich erkennbar, daß beide Agenzien allein im Vergleich zur Kombination einen deutlich geringeren virusinaktivierenden Effekt auf HIV-1 ausüben. Dagegen ist aus Tabelle 1 ersichtlich, daß der Effekt der Kombination ein synergistischer ist, zumal die Inaktivierungskinetik der Kombination aus Polyether und chaotropem Agens wesentlich rascher verläuft als die Addition der Kinetik, die durch die einzelnen Agenzien erreicht wird.

## Patentansprüche

1. Verfahren zur Herstellung eines biologischen Präparates unter Inaktivierung von Viren der Gruppe der großen, membranumhüllten RNA-Viren, kleinen, membranumhüllten RNA-Viren, und membranumhüllten DNA-Viren, wobei die biologische Aktivität des Präparates im wesentlichen beibehalten wird, **gekennzeichnet durch** eine Behandlung des Präparates zur Inaktivierung von infektiösen Agentien in Gegenwart eines Polyethers und eines chaotropen Agens ausgewählt aus der Gruppe Thiocyanate, Harnstoff und Guanidiniumsalze.

2. Verfahren nach Anspruch 1, **dadurch gekennzeichnet, dass** eine Lösung des Präparates behandelt wird.

3. Verfahren nach Anspruch 1 oder 2, **dadurch gekennzeichnet, dass** eine physikalisch-chemische oder eine chemische Behandlung zur Inaktivierung von infektiösen Agenzien vorgenommen wird.

4. Verfahren nach einem der Ansprüche 1 bis 3, **dadurch gekennzeichnet, dass** als Polyether ein Polyhydroxyether verwendet wird.

5. Verfahren nach Anspruch 4, **dadurch gekennzeichnet, dass** als Polyhydroxyether Polyethylenglykol oder Polypropylenglykol verwendet wird.

6. Verfahren nach einem der Ansprüche 1 bis 5, **dadurch gekennzeichnet, dass** die Inaktivierung von infektiösen Agenzien in Anwesenheit eines niedermolekularen Polyethylenglykols, ausgewählt aus der Gruppe PEG 200, PEG 300, PEG 400, PEG 600, PEG 900 und PEG 1000, erfolgt.

7. Verfahren nach einem der Ansprüche 1 bis 6, **dadurch gekennzeichnet, dass** die Inaktivierung von infektiösen Agenzien in Gegenwart eines Polyethers in einer Konzentration von 5 bis 30 Gew.-% erfolgt, wobei Proteine nicht präzipitiert werden.

8. Verfahren nach einem der Ansprüche 1 bis 7, **dadurch gekennzeichnet, dass** als Thiocyanat Ammonium-, Natrium- oder Kaliumthiocyanat verwendet wird.

9. Verfahren nach einem der Ansprüche 1 bis 7, **dadurch gekennzeichnet, dass** als Guanidiniumsalz Guanidiniumhydrochlorid verwendet wird.

10. Verfahren nach einem der Ansprüche 1 bis 9, **dadurch gekennzeichnet, dass** das chaotrope Agens in einer Konzentration von 0,1 bis 2 M eingesetzt wird.

11. Verfahren nach einem der Ansprüche 1 bis 8, **dadurch gekennzeichnet, dass** als chaotropes Agens ein Thiocyanat in einer Konzentration von 0,1 bis 2 M eingesetzt wird.

12. Verfahren nach einem der Ansprüche 1 bis 11, **dadurch gekennzeichnet, dass** die Behandlung bei einer Temperatur im Bereich von 20 bis 60°C durchgeführt wird.

13. Verfahren nach einem der Ansprüche 1 bis 12, **dadurch gekennzeichnet, dass** die Behandlung während einer Dauer von 1 bis 10 h durchgeführt wird.

14. Verfahren nach einem der Ansprüche 1 bis 13, **dadurch gekennzeichnet, dass** das biologische Präparat ein Plasmaprotein umfassend ein Immunglobulin ist.

15. Verfahren nach einem der Ansprüche 1 bis 13, **dadurch gekennzeichnet, dass** als biologisches Präparat ein Faktor der Gerinnung, Fibrinolyse oder Thrombolyse oder eine Inhibitörsubstanz derselben als Plasma protein vorliegt.

16. Verfahren nach einem der Ansprüche 1 bis 15, **dadurch gekennzeichnet, dass** die Behandlung unter Bedingungen durchgeführt wird, unter denen die biologische Aktivität des Präparates zu mindestens 50%, vorzugsweise mindestens 80%, am meisten bevorzugt mindestens 90% erhalten wird.

17. Verfahren nach einem der Ansprüche 1 bis 16, **dadurch gekennzeichnet, dass** die Inaktivierung in Gegenwart des Polyethers und des chaotropen Agens durch eine physikalische, physikalisch-chemische oder chemische Behandlung mit der Ausnahme einer Detergensbehandlung erfolgt.

## Claims

1. Process for producing a biological preparation with inactivation of viruses of the group of large, membrane-enveloped RNA viruses, small, membrane-enveloped RNA viruses, and membrane-enveloped DNA viruses, wherein the biological activity of the preparation is essentially retained, **characterised by** treatment of the preparation for inactivating infectious agents in the presence of a polyether and a chaotropic agent selected from the group thiocyanates, urea and guanidinium salts.

2. Process according to claim 1, **characterised in that** a solution of the preparation is treated.

3. Process according to claim 1 or 2, **characterised in that** a physico-chemical or a chemical treatment is carried out to inactivate infectious agents.

4. Process according to one of claims 1 to 3, **characterised in that** a polyhydroxyether is used as polyether.

5. Process according to claim 4, **characterised in that** polyethylene glycol or polypropylene glycol is used as polyhydroxyether.

6. Process according to one of claims 1 to 5, **characterised in that** the inactivation of infectious agents takes place in the presence of a low-molecular polyethylene glycol, selected from the group PEG 200, PEG 300, PEG 400, PEG 600, PEG 900 and PEG 1000.

7. Process according to one of claims 1 to 6, **characterised in that** the inactivation of infectious agents takes place in the presence of a polyether in a concentration from 5 to 30 wt.%, wherein proteins are not precipitated.

8. Process according to one of claims 1 to 7, **characterised in that** ammonium thiocyanate, sodium thiocyanate or potassium thiocyanate is used as thiocyanate.

9. Process according to one of claims 1 to 7, **characterised in that** guanidinium hydrochloride is used as guanidinium salt.

10. Process according to one of claims 1 to 9, **characterised in that** the chaotropic agent is used in a concentration from 0.1 to 2 M.

11. Process according to one of claims 1 to 8, **characterised in that** a thiocyanate in a concentration from 0.1 to 2 M is used as chaotropic agent.

12. Process according to one of claims 1 to 11, **characterised in that** the treatment is carried out at a temperature in the range from 20 to 60°C.

13. Process according to one of claims 1 to 12, **characterised in that** the treatment is carried out for a period of 1 to 10 hours.

14. Process according to one of claims 1 to 13, **characterised in that** the biological preparation is a plasma protein including an immunoglobulin.

15. Process according to one of claims 1 to 13, **characterised in that** as biological preparation, a factor of coagulation, fibrinolysis or thrombolysis or an inhibitor substance of the same is present as plasma protein.

16. Process according to one of claims 1 to 15, **characterised in that** the treatment is carried out under conditions, under which the biological activity of the preparation is at least 50 % retained, preferably at least 80 %, at the most preferably at least 90 %.

17. Process according to one of claims 1 to 16, **characterised in that** the inactivation takes place in the presence of polyether and chaotropic agent by a physical, physico-chemical or chemical treatment with the exception of detergent treatment.

## Revendications

1. Procédé de fabrication d'une préparation biologique avec inactivation de virus du groupe de grands virus ARN enveloppés par une membrane, de petits virus ARN enveloppés par une membrane et de virus ADN enveloppés par une membrane, l'activité biologique de la préparation étant pratiquement conservée, **caractérisé par** un traitement de la préparation pour obtenir l'inactivation des agents infectieux, en présence d'un produit polyéther et d'un agent chaotropène, sélectionné dans le groupe des thiocyanate, de l'urée et des sels de guanidinium.

2. Procédé selon la revendication 1, **caractérisé en ce que** l'on traite une solution de la préparation.

3. Procédé selon la revendication 1 ou 2, **caractérisé en ce qu'**un traitement physique-chimique ou chimique est effectué pour inactiver des agents infectueux.

4. Procédé selon l'une des revendications 1 à 3, **caractérisé en ce qu'**on utilise du polyhydroxyéther comme polyéther.

5. Procédé selon la revendication 4, **caractérisé en ce qu'**on utilise du polyéthylèneglycol ou du polypropylèneglycol comme polyhydroxyéther.

6. Procédé selon l'une des revendications 1 à 5, **caractérisé en ce que** l'inactivation d'agents infectueux s'effectue en présence d'un polyéthylèneglycol à faible poids moléculaire, sélectionné parmi le groupe des PEG 200, PEG 300, PEG 400, PEG 600, PEG 900 et PEG 1000.

7. Procédé selon l'une des revendications 1 à 6, **caractérisé en ce que** l'inactivation des agents infectueux s'effectue en présence d'un polyéther, en une concentration de 5 à 30 % en poids, les protéines n'étant pas précipitées.

8. Procédé selon l'une des revendications 1 à 7, **caractérisé en ce que** l'on utilise comme thiocyanate du thiocyanate d'ammonium, de sodium ou de potassium.

9. Procédé selon l'une des revendications 1 à 7, **caractérisé en ce que** l'on utilise comme sels de guanidinium de l'hydrochlorure de guanidinium.

10. Procédé selon l'une des revendications 1 à 9, **caractérisé en ce que** l'agent chaotrope est utilisé en une concentration de 0,1 à 2 M.

11. Procédé selon l'une des revendications 1 à 8, **caractérisé en ce que** l'on utilise comme agent chaotrope un thiocyanate en une concentration de 0,1 à 2 M.

12. Procédé selon l'une des revendications 1 à 11, **caractérisée en ce que** le traitement est effectué à une température dans la plage de 20 à 60° C.

13. Procédé selon l'une des revendications 1 à 12, **caractérisé en ce que** le traitement est effectué pendant une durée de 1 à 10 heures.

14. Procédé selon l'une des revendications 1 à 13, **caractérisé en ce que** la préparation biologique est une plasmaprotéine comprenant une immunoglobuline.

15. Procédé selon l'une des revendications 1 à 13, **caractérisé en ce qu'**on a comme préparation biologique, un facteur de coagulation, fribrinolyse ou thrombolyse ou une substance inhibitrice, sous forme de plasmaprotéine.

16. Procédé selon l'une des revendications 1 à 15, **caractérisé en ce que** le traitement est effectué dans des conditions dans lesquelles l'activité biologique de la préparation est obtenue pour au moins 50 %, de préférence au moins 80 %, de la manière la mieux préférée d'au moins 90 %.

17. Procédé selon l'une des revendications 1 à 16, **caractérisé en ce que** l'inactivation s'effectue en présence du polyéther et de l'agent chaotrope par un traitement physique, physico-chimique ou chimique, à l'exception d'un traitement par détergents.
